# NEUE EUROPÄISCHE PATENTSCHRIFT

(11) **EP 0 218 928 B2**
(45) Veröffentlichungstag und Bekanntmachung des Hinweises auf die Entscheidung über den Einspruch: **18.01.1995**
(45) Hinweis auf die Patenterteilung: 05.09.1990
(21) Anmeldenummer: 86112636.5
(22) Anmeldetag: 12.09.1986
(51) Int. Cl.: A61K 9/20, A61K 9/50, A61K 9/52

(54) **Pellets mit irregulärer Oberfläche, Verfahren zu deren Herstellung und Tabletten enthaltend diese Pellets**
Pellets with irregular surface, process for their preparation and tablets containing them
Pellets à surface irregulière, procédé de préparation et comprimés les contenant

(30) Priorität: 13.09.1985 DE 3532692
(43) Veröffentlichungstag der Anmeldung: 22.04.1987
(73) Patentinhaber: BOEHRINGER MANNHEIM GMBH, 68298 Mannheim (DE)
(72) Erfinder: Demmer, Fritz, Dr. rer. nat., D-6945 Hirschberg-Leutershausen (DE); Groppenbächer, Gregor, Dr. rer. nat., D-6805 Heddesheim (DE); Stemmle, Berhold, Dr. rer. nat., D-6832 Hockenheim (DE)

(56) Entgegenhaltungen:
- FR-A- 2 084 199
- FR-A- 2 559 389
- CHEMICAL ABSTRACTS, Band 77, Nr. 14, 2. Oktober 1972, Seite 310, Zusammenfassung 92800b, Columbus, Ohio, US; Y. KAWASHIMA et al.: "Particle properties of spray-dried and agglomerated products of salicylic acid and sodium salicylate", & ZAIRYO 1972, 21(225), 557-61
- CHEMICAL ABSTRACTS, Band 97, Nr. 26, Dezember 1982, Seite 445, Zusammenfassung 222958k, Columbus, Ohio, US; & RO-A-71 837 (INTREPRINDEREA DE MEDICAMENTE, BUCURESTI) 20-08-1981
- PHARMACEUTICAL TECHNOLOGY, June 1985, pp. 52-60, A. Mehta and D. Jones: "Coated pellets under the microscope"
- Arzneiformenlehre, Ein Lehrbuch für Pharmazeuten, 1976, S.77-80
- Arzneiformenlehre, Ein Lehrbuch für Pharmazeuten, 1985, S. 83

## Beschreibung

Gegenstand der vorliegenden Erfindung sind Tabletten als pharmazeutische Zubereitung mit definierter, vorzugsweise retardierter Freisetzung des Wirkstoffes sowie ein entsprechendes Herstellungsverfahren.

In neuerer Zeit wird zunehmend versucht, die Freisetzungsgeschwindigkeit von Arzneimitteln besser zu steuern. Neben den Retardzubereitungen, bei denen es darauf ankommt, die Freisetzung über längere Zeiträume (2-8 h) auf einem möglichst gleichmäßigen Niveau zu halten, gewinnt das Problem einer möglichst kurzfristigen, definierten Freisetzung an Bedeutung.

Herkömmliche Tablettenzubereitungen, bei denen die pulverförmigen Wirk- und Hilfsstoffe zunächst feucht oder trocken granuliert und anschließend unter Zusatz von Spreng- und Gleitmitteln tablettiert werden, haben sich als nicht genügend zuverlässig erwiesen. Dies beruht darauf, daß bei der Granulierung Teilchen sehr unterschiedlicher Größe (Durchmesser 0,1-2 mm) entstehen, die folglich auch eine unterschiedliche Auflösungsgeschwindigkeit besitzen, und ferner beim Verpressen zu Tabletten trotz des Sprengmittels die Granulen aufgrund ihrer unregelmäßigen Form teilweise so stark miteinander verzahnen, daß die Tablette beim Einbringen in Wasser nicht mehr vollständig in die Granulen zerteilt wird, sondern erheblich größere Komplexe, die sich langsamer lösen, übrig bleiben.

In neuerer Zeit werden deshalb für freisetzungsgesteuerte oder Retardzubereitungen häufig wirkstoffhaltige Pellets enwickelt, die anschließend in Steckkapsein abgefüllt werden.

Das Wort Pellet entstammt der englischen Sprache und läßt sich am ehesten mit Kügelchen übersetzen. Pellets stellen eine feste Arzneiform dar, die nach ihrer Geometrie definiert ist, während Herstellungsverfahren und Verwendungszweck variabel sind. Von den Granulen unterscheiden sich Pellets durch ihre fast ideale Kugelform und ihre glatte Oberfläche und von anderen kugelförmigen Arzneimiteln durch ihre Größe (normalerweise unter 1 mm Durchmesser).

Die Darreichungsform der in Kapseln abgefüllten Pellets soll im Vergleich zu den Retardformen, die mit einer Tablette erreicht werden können, z.B. Gerüst-, Wachs- oder Polymermatrix oder Hydrogelbarriere den Vorteil aufweisen, daß sich die Einzelpellets in einer sehr engen Größenverteilung herstellen lassen und nach Einnahme und Auflösung der Kapsel über den gesamten Mageninhalt verteilen, von wo sie durch schubweisen Weitertransport des Mageninhalts in die resorptionsfähigen oberen Darmabschnitte gelangen. Aufgrund dieses Verhaltens soll eine gleichmäßigere, sichere Resorption im Vergleich zu Tablettenzubereitungen gewährleistet sein.

Neben speziellen, nicht für alle Anwendungszwecke nutzbaren Methoden sind es die folgenden abgewandelten Granulierverfahren, die für die Herstellung der kugelfömigen Pellets angewandt werden.

Eine Möglichkeit der Herstellung von Pellets geht von herkömmlichen Abbau-Granulierverfahren aus. Diese liefern in der Regel keine ideal kugelförmigen Teilchen, so daß weitere Operationen wie Zerkleinerung. Formung und Klassierung nötig sind. Als erfolgreich hat sich die Kombination von Press- oder Lochstreifengranulation mit sog. Rundungsmaschinen herausgestellt. Das zylindrische Granulat wird in diesen Geräten durch plastische Verformung und Abrieb zu runden Teilchen transformiert. Nachteilig ist ein Materialverlust als Staub, der jedoch ggf. durch Recycling kompensiert werden kann. Weiterhin weisen derartige Produkte meist eine begrenzte mechanische Festigkeit auf, denn die Granulatentstehung durch Krustenbildung zwischen Pulverpartikeln bedingt eine relativ hohe Porosität, die nur zum Teil während des Rundungsprozesses durch Verdichtung vermindert wird.

Auch die Wirbelschicht-Technologie kann bei entsprechender Verfahrensführung erfolgreich zur Pelletherstellung eingesetzt werden. Durch Einbau eines Rotors wird dabei eine Gutbewegung erreicht, die die Entstehung kugelförmiger Partikel begünstigt. Vorteil dieser Aufbau-Granulation ist, daß weitere Produktionsschritte (Trockung, Coating) in demselben Gerät durchgeführt werden können. In den meisten Fällen ist jedoch eine Klassierung aufwendig, denn das erhaltene Teilchengrößenspektrum ist recht breit; auch die Partikelform und -porosität ist selten optimal.

Ebenso geeignet für die Pelletfabrikation sind die verschiedenen Dragierverfahren, da wegen der Rolliervorgänge direkt runde Teilchen erhalten werden. Est durch Klassierung werden die gewünschten Korngrößenbereiche erhalten. Dabei kommen meist Siebverfahren zur Anwendung. Da die Rohstoffe als Losung aufgebracht und durch Trocknung und Kristallisation zur Drageedecke umgewandelt werden. erhält man Pellets hoher Dichte und großer mechanischer Festigkeit. Ein Nachteil besteht in der thermischen Belastung des Materials bei den notwendigen Trocknungsschritten, die jedoch durch geeignete Verfahrensführung vermindert werden kann.

Ferner können Pellets durch Sprüherstarrung entsprechender Schmelzen erzeugt werden. Erforderliche Randbedingungen sind normalerweise die rasche Erstarrung der Schmelze nach dem Zerstäuben, was durch die Wahl der Hilfsstoffe gesteuert werden kann, sowie die gleichmäßige Verteilung und Temperaturbeständigkeit des Wirkstoffs in der Schmelze.

Für den Hersteller wirkstoffhaltiger Pellets ergeben sich zwei Alternativen: Eine Möglichkeit ist, daß die Pellets in ihrer gesamten Masse Wirkstoff enthalten. Dies scheint bei hochdosierten Arzneistoffen oft unumgänglich. Nachteilig ist, daß die Substanzen schon vor Beginn der Arzneiformung an dem Vefahrensprozeß teilnehmen. So geht ein Teil als Abrieb verloren, durch Trocknungsvorgänge erfolgt eine thermische Belastung, entsprechende Sicherheitsvorkehrungen sind nötig und der Wirkstoffgehalt ist bereits analytisches Kriterium für die Zwischenprodukte.

Bei der Verarbeitung schon in geringen Mengen wirksamer Arzneistoffe ergibt sich eine elegantere Möglichkeit- in der Verwendung von Trägerpellets aus indifferenten Rohstoffen von pharmazeutischer Qualität, auf die eine Drageedecke aufgebracht wird, die den Wirkstoff enthält (vgl. D. Werner, Pharma Int. 1984, S. 70 ff). Durch Mischen von Pellets verschiedener Zusammensetzung können Wirkstoffkombinationen auch von sonst miteinander unverträglichen Wirkstoffen hergestellt werden. Durch Mischen von "retardierten Pellets" mit Pellets normaler Freisetzung läßt sich zusätlich die Freisetzungsgeschwindigkeit optimal steuern.

Ein Nachteil der Darreichungsform "Pellets in Kapseln" ist der im Vergleich zu Tabletten relativ hohe Herstellungspreis (Kosten der Leerkapseln, geringere Abfülleistung der Kapselmaschine im Vergleich zu einer Tablettenmaschine).

Bei hochdosierten Wirkstoffen hat die Darreichungsform Pellets in Kapseln den Nachteil, daß für die erforderliche Anzahl von Pellets aufgrund der Schüttung ein großes Volumen benötigt wird, was wiederum sehr große Kapseln erforderlich macht, wodurch die gewünschte Patienten-Compliance in Frage gestellt wird. Es wäre deshalb ein vorteilhaftes, rationelles Verfahren, Pellets gegebenenfalls mit weiteren pharmazeutisch üblichen Hilfsstoffen zu tablettieren, wenn gewährleistet ist, daß die Tablette nach Einnahme in den Verdauungssäften in die Einzelpellets zerfällt und dadurch die gewünschten Pelleteigenschatten erhalten bleiben. Prinzipiell ist eine Tablettierung von Pellets möglich. Entsprechende Tabletten weisen jedoch bezüglich der üblichen. Qualitätsparameter von Tabletten schwerwiegende Nachteile auf. Es werden Tabletten mit ungenügender Härte und hoher Friabilität erhalten, die z.B. beim Ausdrücken aus Blisterstreifen und bei der Verarbeitung teilweise bereits zerbrechen bzw. zerbröseln können.

Werden zur Erzielung einer kompakteren Tablette höhere Preßkräfte angewendet, dann führt dies zu einer Zerstörung der Pelletstruktur, was zur Folge hat, daß die Tablette nicht mehr in die einzelnen Pellets zerfällt, sondern wieder größere Aggregate gebildet werden, so daß die erwünschte geleichmaßige Wirkstoff-Abgabe bei der Magen-Darm-Passage nicht mehr gewährleistet ist.

Der Erfindung liegt daher die Aufgabe zugrunde, eine Arzneimittelzubereitung mit definierter, insbesondere retardierter Freisetzung des Wirkstoffes zur Verfügung zu stellen, welche einerseits kostengünstig herzustellen ist und andererseits auch für hohe Wirkstoffkonzentrationen ein verhältnismäßig geringes Volumen hat, um die Einnahme zu erleichtern.

Die Aufgabe wird durch die in Anspruch 1 definierte Erfindung gelöst.

Dem Fachmann ist es (beispielsweise aus dem Buch "Arzneiform-Lehre" von P. H. List, Wissenschaftliche Verlagsgesellschaft Stuttgart, 1976) bekannt, daß die mechanische Festigkeit von aus Granulaten hergestellten Tabletten dadurch gefördert wird, daß die Granulatkörner aufgrund ihrer unebenen, rauhen und gezackten Oberfläche beim Verpressen ineinander verzahnen und verkeilen. Zugleich ist dem Fachmann bewußt, daß die Tablettierung von Granulaten mit einer Zerstörung der Granulatpartikel verbunden ist. In dem genannten Buch wird im einzelnen erläutert, daß es beim Pressen von Tabletten stets zu irreversiblen Formänderungen und Brüchen im Preßgut kommt, wobei die entstehenden kleineren Teilchen die vorhandenen Lücken auffüllen und dadurch zum Zusammenhalt der Tablette beitragen. Bei einer freisetzungsgesteuerten Arzneiform auf Basis von Pellets ist es dagegen unabdingbar, daß die Pelletstruktur in der Tablette unzerstört bleibt, weil nur dadurch die definierte Freisetzung des Wirkstoffes in der Magen-Darm-Passage gewährleistet werden kann.

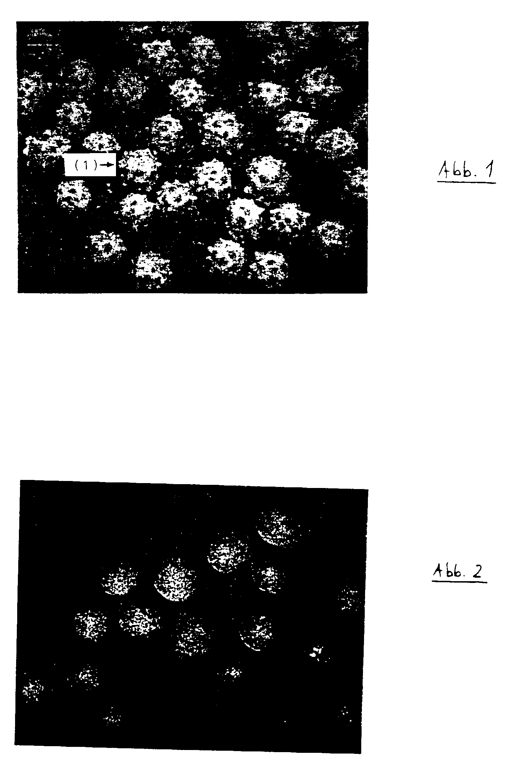

Die für die Erfindung geeigneten Pellets mit einer Vielzahl von Erhebungen in Form von Spitzen und Vorsprüngen lassen sich nach zwei Fertigungsvarianten herstellen:
Nach üblichen Methoden hergestellte Pellets, die eine kugelige oder eiförmige Gestalt und eine glatte Oberfläche besitzen und häufig mit einem diffusionsverzögernden überzug versehen sind, der etwaige Unebenheiten zusätzlich glättet, werden mit einer möglichst konzentrierten Lösung oder Suspension eines gut kristallisierenden, pharmakologisch verträglichen Hilfsstoffs dragiert, wobei durch gelichzeitige Trocknung das Lösungsmittel so rasch verdampft wird, daß die Lösung sich nicht über die Oberfläche der Pellets verteilt und einen glatten Film bilden kann, sondern örtlich unter Ausbildung von makroskopisch sichtbaren Spitzen und Vorsprüngen eintrocknet und kirstallisiert, (vgl. (1) in Abb. 1). Es wird daruaf verwiesen, daß die Bezugszeichen hinter den in den Ansprüchen genannten Merkmalen lediglich zum besseren Verständenis der Ansprüche aufgenommen worden sind. Saccharose, Lactose und ähnliche Stoffe können zu diesem Zweck einzeln oder kombiniert vorteilhaft verwendet werden, wobei üblicherweise mit wässrigen Lösungen gearbeitet wird. Sollten die Pellets mit Wasser unverträglich sein, kann natürlich auch mit einem organischen Lösungsmittel gearbeitet werden. Gleitmittel, Sprengmittel, Farbstoffe und andere Hilfsstoffe, ohne "weichmachende Eigenschaften", können dieser Nachdragierlösung bzw. -suspension natürlich ebenfalls zugefügt werden. "Weichmachende" Stoffe, wie z.B. niedermolekulare Polyethylenglykole (MG unter 1 000) oder niedrigschmelzende Fette und Wachse, sollten nicht oder nur in geringer Menge zugesetzt werden, da sie sonst während und nach der Trocknung eine Glättung und Verteilung des Überzuges bewirken. Soweit die Pellets, insbesondere ein daruaf angebrachter Diffusionsfilm, als Haftgrund für den irregulären Überzug nicht geeignet ist, kann zunächst ein Haftvermittler. bspw. eine Polyvinylpyrrolidon, Polyvinylakohol, Gelatine, Gummiarabikum oder ähnliche Stoffe enthaltende Lösung, aufgetragen werden.

Bei Aufbaupellets, die durch Schichtaufbau aus einer Wirkstoffsuspension auf Neutralpellet erzeugt werden, ergibt sich die zusätzliche Möglichkeit, die Trocknungsbedingungen zu verstärken bzw. die Zusatzgeschwindigkeit und/oder Konzentration und/oder Zusammensetzung der Dragiersuspension zu verändern und dadurch ein unregelmäßiges Auskristallisieren der Wirkstoffrezeptur selbst zu erreichen. Der normalerweise beim Dragieren bzw. Pellettieren als "Kunstfehler" und unerwünschter Effekt auftretende "orange peel"-Effekt wird dadurch absichtlich nicht nur erreicht, sondern so weit gesteigert, daß sich eine zur Verzahnung beim Tablettieren ausreichend irreguläre Oberfläche ausbildet.

Wie bei der nachträglichen Beschichtung müssen die Wirkstoffe und Hilfsstoffe gut kristallisieren und die Rezeptur darf keine "weichmachenden" substanzen enthalten. Ein nachträgliches Überziehen mit einem Diffusionsfilm ist bei solchen Präparaten allerdings nicht möglich, da einerseits die Oberfläche der Pellets dadurch wieder geglättet würde und andererseits ein gleichmäßiger Filmüberzug nicht möglich ist.

Ein Unterscheidungsmerkmal zwischen Pellets mit glatter Oberfläche und solchen mit irregulärer Oberfläche bildet neben dem mikroskopisch Feststellbaren verschiedenem Erscheinungsbild das unterschiedliche Schüttgewicht, das für Pellets mit irregulärer Oberfläche um bis zu 30% geringer ist als das von regulären Pellets der gleichen Masse und der gleichen Größenverteilung.

In den Abbildungen 1 und 2 sind normal runde, glatte Pellets gemäß dem Stand der Technik (Abb. 2) und erfindungsgemäß erhaltene irreguläre Pellets (Abb. 1) abgebildet. Beide Abbildungen sind 10:1 vergrößert. Man erkennt in Abb. 1, daß die irregulären Pellets eine Vielzahl von Erhebungen (1) auf dem kugel- oder ellipsoidförmigen Grundkörper aufweisen, während in Abb. 2 die vergleichbaren Grundkörper glatt sind und keine Vorsprünge oder "Unstetigkeiten" besitzen. Aus diesen irregulären Pellets lassen sich erfindungsgemäß unter Zusatz üblicher Tablettier-Hilfsstoffe, wie Gleitmittel, Sprengmittel, Bindemittel etc., harte, abriebfeste, beim Einbringen in Wasser wieder in die Pellets zerfallende Tabletten pressen.

Der Pressdruck beträgt üblicherweise 4.905.10⁷ N/m² - 29.43.10⁷ N/m² (0`5-3 t/cm²), die erreichte Tablettenhärte 150-300 N. Unter gleichen Bedingungen verarbeitete glatte Pellets besitzen dagegen eine Härte von höchstens 100 N und sind so weich, daß sie bereits bei der Bearbeitung zerbröckeln.

In den folgenden Beispielen wird die Erfindung näher ausgeführt. Weitere Anwendungsmöglichkeiten kann der Fachmann ohne weiteres ableiten.

### Beispiel 1

### 1a) Herstellung von Bezafibrat-Pellets mit irregulärer Oberfläche

Apparat: Zwiebelförmiger Dragierkessel mit einem Fassungsvermögen von 10 kg
Einlage: 1 000 g Placebo-Minipellets mit einem Durchmesser von 0,5-0,6 mm
Trocknungsluft: 80 m³/h und 50°C
Dragiersuspension:

| | |
|---|---|
| Wirkstoff (Bezafibrat): | 23,16% |
| Polyacrylharzdispersion in Wasser (Eudragit® E 30 D)(30 %ig): | 12,47% |
| Polyethylenglycol (Macrogol® 6000)(50 %ige wässrige Lösung): | 1,50% |
| Saccharose | 1,98% |
| Hydroxypropylmethylcellulose als 20 %ige wässrige Lösung | |
| (Pharmacoat® 603) | 1,85% |
| Entmineralisiertes Wasser | 59,04% |
| | 1̅0̅0̅,̅0̅0̅%̅ |

Herstellung der Suspension: Alle Bestandteile, außer der Dispersion Eudragit® E 30 D, werden in einem geeigneten Behälter mit einem Intensivmischer homogenisiert und anschließend Eudragit® E 30 D gleichmäßig eingerührt.

### Ausführung:

Durch Einsprühen von oben oder mit Hilfe eines Tauchrohrs in die in den Dragierkessel in Bewegung gehaltenen Pellets werden 30 kg der angegebenen Suspension unter gleichzeitiger Trocknung aufgesprüht. Die Zugabegeschwindigkeit beträgt zu Beginn des Verfahrens ca. 520 g/h und wird für die restlichen 90% der Suspension auf ca. 700 g/h erhöht. Die Pellets werden noch 10 min nachgetrocknet und dann entnommen. Es entstehen Pellets mit einem Wirkstoffgehalt von 63,0% und einer irregulären Oberfläche (vgl. Abb. 1). Der Durchmesser beträgt 0,9-1,2 mm und liegt damit in den für Pellets üblichen engen Grenzen. Das Schüttgewicht der Pellets liegt im Bereich von 60-70 g/100 ml.

### 1b) Bezafibrat-400 mg-retard-Tabletten

| Zusammensetzung | mg/Stück | 10 000 Stück |
|---|---|---|
| I-Bezafibrat-Pellets mit irregulärer Oberfläche entsprechend (Beispiel 1a) | 700,0 mg | 7 000 g |
| II-Microcristalline Cellulose | 48,0 mg | 480 g |
| Natriumcarboxymethylstärke | 40,0 mg | 400 g |
| Siliciumdioxid, hochdispers | 4,0 mg | 40 g |
| Magnesiumstearat | 4,0 mg | 40 g |
| | 7̅9̅6̅,̅0̅ m̅g̅ | 7̅.̅9̅6̅0̅ g̅ |

Zumischung II durch ein Sieb mit 0,8 mm Maschenweite geben und mit den Pellets I mischen.

Auf einer geeigneten Tablettiermaschine verpresen (Preßdruck 4.905.10⁸ N/m² entsprechend, ca. 5 t/cm²). Format 8 mm × 17 mm, oblong.

| | |
|---|---|
| Härte: | 200 N |
| Abrieb: | 0,2% |

### Beispiel 2

### 2a) Herstellung von Bezafibrat-Pellets mit glatter Oberfläche

In einem Drageekessel analog zu Beispiel 1 wird die gleiche Rezeptur wie in Beispiel 1 verarbeitet, bis auf den Anteil an Polyacrylharz, welches durch eine entsprechende Menge Wasser und das Macrogol® 6000 durch Macrogol® 400 ersetzt wurde. Die Auftragsgeschwindigkeit wurde über die ganze Zeit konstant bei ca. 800 g/h gehalten. Die Pellets werden 30 min nachgetrocknet und während dieser Zeit im Kessel weiter umgewälzt. Es entstehen praktisch kugelförmige Pellets mit glatter Oberfläche (Durchmesser 0,8-1,0 mm).

Um eine Retardierung der Wirkstoffe zu erzielen, wurden diese Pellets im gleichen Kessel anschließend mit 40 mg Eudragit® E 30 D (30% wässrige Lösung) entsprechend 12 mg Trockenstoff/g Pellets überzogen und getrocknet. Es entstehen glatte, gleichförmige Pellets (vgl. Abb. 2). Das Schüttgewicht der Pellets liegt im Bereich von 70-80 g/100 ml.

### 2b) Bezafibrat-400 mg-retard-Tabletten

| Zusammensetzung | mg/Stück | 10 000 Stück |
|---|---|---|
| I-Bezafibrat-Pellets mit Diffusionsfilm und regulärer Oberfläche entspechend (Beispiel 2a) | 636,0 mg | 6 360 g |
| II-Microcristalline Cellulose | 116,0 mg | 1 160 g |
| Natriumcarboxymethylstärke | 40,0 mg | 400 g |
| Siliciumdioxid, hochdispers | 4,0 mg | 40 g |
| Magnesiumstearat | 4,0 mg | 40 g |
| | 8̅0̅0̅,̅0̅ m̅g̅ | 8̅ 0̅0̅0̅ g̅ |

Herstellung wie in Beispiel 1 beschrieben.
Härte, maximal möglich: 100 N
Abrieb: 100% (alle Tabletten zerbrochen)

### Beispiel 3

- Apparat:: Zwiebelförmiger Dragierkessel mit einem Fassungsvermögen von 10 kg
- Einlage:: 5 000 g Pellets mit Diffusionsfilm und regulärer Oberfläche, gemäß Beispiel 2a)
- 1. Stufe: (Haftgrund): Eine Lösung von 20 g Polyvinylpyrrolidon (Kollidon® 25) in 80 g Wasser wird in 2 Portionen aufgegossen und jeder Aufguß mit einer Pulvermischung, bestehend aus 10 g Saccharose und 10 g Talkum, abgestäubt. Anschließend wird 10 Minuten mit einem Luftstromm von 1,33 m³/min und einer Temp. von 50°C getrocknet.
- 2. Stufe. (Deckschicht): Auf die nach 1. vorbehandelten Pellets werden anschließend 200 g Suspension nachfolgender Zusammensetzung kontinuierlich während 1 Stunde aufgesprüht, entweder von oben oder mit dem Tauchrohrverfahren. Die Trocknungsbedingungen 80 m³/50°C im Dragierkessel sind so gewählt, daß der Prozeß "trocken" abläuft. Es entstehen Pellets mit irregulärer Oberfläche.

### Zusammensetzung der Suspension:

| | |
|---|---|
| Saccharose: | 48,00% |
| Kaolin: | 12,00% |
| Macrogol® 6 000: | 5,00% |
| Entmin. Wasser: | 35,00% |
| | 1̅0̅0̅,̅0̅0̅%̅ |

### Herstellung der Suspension:

Die Saccharose und as Macrogol® 6 000 werden im entmineralisierten Wasser bei einer Temperatur von 25°C gelöst. In diese Lösung wird das Kaolin eingerüht und die Suspension homogenisiert.

Die Pellets werden entsprechend Beispiel 1 zu Tabletten verpreßt.

Die Härte der Tabletten beträgt 173 N, der Abrieb liegt bei 0,15%.

### In-vitro-Auflösungsrate

Die in-vitro-Auflösungsrate der Tabletten entspricht, wie aus der folgenden Tabelle hervorgeht, praktisch der Auflösungsrate der Pellets, wenn man berücksichtigt, daß die Tablette innerhalb von ca. 30 Minuten in die Einzelpellets zerfällt. Die Zerfallzeit kann durch Art und Menge der Zumischung II beliebig variiert werden.

### Auflösungsgeschwindigkeit in-vitro [%]

| Zeit in h | Pellets Beisp. 1a | Tabletten Beisp. 1b | Pellets Beisp. 2 | Pellets Beisp. 3a | Tabletten Beisp. 3b |
|---|---|---|---|---|---|
| 1,0 | 35 | 21 | 49 | 61 | 64 |
| 2,0 | 53 | 50 | 71 | 82 | 84 |
| 3,0 | 63 | 66 | 80 | 94 | 92 |
| 4,0 | 69 | 77 | 85 | 96 | 95 |
| 5,0 | 80 | 80 | 92 | 103 | 97 |

## Patentansprüche

1. Tabletten als pharmazeutische Zubereitung mit definierter Wirkstofffreisetzung, **dadurch gekennzeichnet**, daß
sie Pellets mit einer unregelmäßigen Oberfläche mit einer Vielzahl von Erhebungen in Form von makroskopisch sichtbaren Spitzen und Vorsprüngen auf einem kugeligen Grundkörper enthalten, so daß beim Tablettiervorgang bei niedrigen Preßdrucken eine oberflächliche Verzahnung der Einzelpellets erfolgt und
die Pellets so verpreßt sind, daß die Pelletsstruktur in der Tablette unzerstört ist und die Tablette nach der Einnahme in die einzelnen Pellets zerfällt, so daß eine gleichmäßige Wirkstoff-Abgabe bei der Magen-Darm-Passage gewährleistet ist.

2. Tabletten gemäß einem der Ansprüche 1 oder 2, **dadurch gekennzeichnet**, daß die Pellets als Wirkstoff Bezafibrat enthalten.

3. Tabletten gemäß einem der Ansprüche 1 oder 2, **dadurch gekennzeichnet**, daß die Pellets einen die Diffusion des Wirkstoffes steuernden Film aufweisen.

4. Tabletten gemäß einem der Ansprüche 1 bis 3, **dadurch gekennzeichnet**, daß die Pellets Aufbaupellets sind.

5. Verfahren zur Herstellung von Pellets für Tabletten gemäß einem der Ansprüche 1 bis 4, **dadurch gekennzeichnet,** daß man zur Erzeugung der irregulären Oberfläche mit Erhebungen in Form von makroskopish sichtbaren Spitzen und Vorsprüngen kugelige Pellets mit einer konzentrierten und gegebenenfalls wirkstoffhaltigen Lösung oder Suspension von gut kristallisierenden, pharmakologisch verträglichen Hilfsstoffen dragiert, wobei unter gleichzeitiger Trocknung das Lösungsmittel so rasch verdampft wird, daß die Lösung oder Suspension unter Ausbildung der Spitzen und Vorsprünge örtlich eintrocknet und kristallisiert.

6. Verfahren gemäß Anspruch 5, **dadurch gekennzeichnet**, daß die wirkstoffhaltige Lösung oder Suspension mehrere Wirkstoffe enthält.

7. Verfahren gemäß Anspruch 5, **dadurch gekennzeichnet**, daß die Lösung oder Suspension Saccharose oder Lactose enthält.

8. Verfahren gemäß Anspruch 5, **dadurch gekennzeichnet**, daß wirkstoffhaltige Pellets zunächst mit einem die Diffusion des Wirkstoffes steuernden Film überzogen werden, bevor die irreguläre Oberfläche aus Spitzen und Vorsprüngen erzeugt wird.

9. Verfahren zur Herstellung von Tabletten nach einem der Ansprüche 1 bis 4, **dadurch gekennzeichnet**, daß man nach einem der Ansprüche 5 bis 8 Pellets herstellt und diese, gegebenenfalls mit üblichen Hilfsstoffen, bei niedrigen Preßdrucken zu Tabletten verpreßt.

10. Verfahren gemäß Anspruch 9, **dadurch gekennzeichnet**, daß der Preßdruck 4,905·10⁷ N/m² bis 29,43·10⁷ N/m² (0,5 bis 3 t/cm²), vorzugsweise 9,81·10⁷ N/m² bis 19,62·10⁷ N/m² (1 bis 2 t/cm²) beträgt.

## Claims

1. Tablets as pharmaceutical composition with defined active material liberation, characterised in that they contain pellets with an irregular surface with a plurality of bumps in the form of macroscopically visible peaks and projections on a spheroidal base body so that, in the case of the tabletting procedure at low pressing forces, a superficial interlocking of the individual pellets takes place and the pellets are so pressed that the pellet structure is not destroyed and the tablets, after taking, break down into the individual pellets so that a uniform release of the active material is ensured in the case of stomach-intestine passage.

2. Tablets according to claim 1, characterised in that the pellets contain bezafibrate as active material.

3. Tablets according to one of claims 1 or 2, characterised in that the pellets have a film controlling the diffusion of the active material.

4. Tablets according to one of claims 1 to 3, characterised in that the pellets are layered pellets.

5. Process for the production of pellets for tablets according to one of claims 1 to 4, characterised in that, for the production of the irregular surface with bumps in the form of peaks and projections, spheroidal pellets are drageed with a concentrated and possibly active material-containing solution or suspension of readily crystallising, pharmacologically compatible adjuvant materials, whereby, with simultaneous drying, the solvent is so rapidly evaporated that the solution or suspension dries and crystallises with formation of peaks and projections.

6. Process according to claim 5, characterised in that the active material-containing solution or suspension contains several active materials.

7. Process according to claim 5, characterised in that the solution or suspension contains saccharose or lactose.

8. Process according to claim 5, characterised in that the active material-containing pellets are first coated with a film controlling the diffusion of the active material before the irregular surface of peaks and projections is produced.

9. Process for the production of pellets according to one of claims 1 to 4, characterised in that one produces pellets according to one of claims 5 to 8 and presses these at low pressing forces to give tablets, possibly with usual adjuvant materials.

10. Process according to claim 9, characterised in that the pressing force amounts to 4.905·10⁷ N/m² to 29.43·10⁷ N/m² (0.5 to 3 t/cm²), preferably 9.81·10⁷ N/m² to 19.62·10⁷ N/m² (1 to 2 t/cm²).

## Revendications

1. Comprimés constituant une préparation pharmaceutique, à libération définie de la substance active, caractérisés en ce qu'ils contiennent des pellets présentant une surface irrégulière avec une multiplicité de proéminences sous forme de pointes et saillies macroscopiques visibles sur un corps de base sphérique, de sorte que lors de l'opération de production des comprimés, il se forme, sous de faibles pressions, un crantage superficiel des pellets individuels, et les pellets sont comprimés de telle façon que leur structure reste intacte à l'intérieur du comprimé et que le comprimé se désagrège, après ingestion, en pellets individuels, de sorte qu'une libération uniforme de la substance organique est assurée dans le tractus gastro-intestinal.

2. Comprimés selon l'une des revendications 1 ou 2, caractérisés en ce que les pellets contiennent du bézafibrate en tant que substance active.

3. Comprimés selon l'une des revendications 1 ou 2, caractérisés en ce que les pellets présentent un film réglant la diffusion de la substance active.

4. Comprimés selon l'une quelconque des revendications 1 à 3, caractérisés en ce que les pellets sont des pellets structurés.

5. Procédé de préparation de pellets pour des comprimés selon l'une quelconque des revendications 1 à 4, caractérisé en ce que pour l'obtention de la surface irrégulière comportant des proéminences sous forme de pointes et saillies, on dragéifie des pellets sphériques avec une solution ou suspension concentrée, contenant éventuellement de la substance active, des adjuvants pharmacologiquement acceptables, ayant une bonne aptitude à la cristallisation, le solvant s'évaporant, pendant le séchage simultané, avec une rapidité telle que la solution ou suspension sèche et cristallise localement en formant les pointes et saillies.

6. Procédé selon la revendication 5, caractérisé en ce que la solution ou suspension contenant de la substance active contient plusieurs substances actives.

7. Procédé selon la revendication 5, caractérisé en ce que la solution ou suspension contient du saccharose ou du lactose.

8. Procédé selon la revendication 5, caractérisé en ce que les pellets contenant de la substance active sont enrobés d'abord avec un film réglant la diffusion de la substance active avant l'obtention de la surface irrégulière formée de pointes et de saillies.

9. Procédé de préparation de comprimés tels que revendiqués dans l'une quelconque des revendications 1 à 4, caractérisé en ce que l'on prépare des pellets selon l'une quelconque des revendications 5 à 8, et on les transforme en comprimés sous de faibles pressions, éventuellement avec des adjuvants usuels.

10. Procédé selon la revendication 9, caractérisé en ce que la pression est comprise entre 4,905 x 10⁷ N/m² et 29,43 x 10⁷ N/m² (0,5 et 3 t/cm²), de préférence 9,81 x 10⁷ N/m² et 19,62 x 10⁷ N/m² (1 et 2 t/cm²).
